# EUROPEAN PATENT APPLICATION

(11) **EP 4 487 836 A1**
(43) Date of publication of application: **08.01.2025**
(21) Application number: 23183012.6
(22) Date of filing: 03.07.2023
(51) Int. Cl.: A61K 9/00, A61K 9/10, A61K 31/365, A61K 47/26, A61K 47/36, A61K 47/38

(54) **AQUEOUS COMPOSITION COMPRISING AVERMECTINS**

(71) Applicant: HWI pharma services GmbH, 76761 Rülzheim (DE)
(72) Inventor: Wissel, Stefan, 76829 Landau (DE); Rischer, Matthias, 60388 Frankfurt (DE); Wissel, Philipp, 75180 Pforzheim (DE); Häberlein, Felix, 53227 Bonn (DE)
(74) Representative: Patentanwälte Ruff, Wilhelm, Beier, Dauster & Partner mbB

(57) **Abstract**

An aqueous composition is provided which comprises at least one surface modifier selected from a group consisting of cellulose derivatives, in particular hydroxypropylmethyl cellulose, methylcellulose and hydroxypropylcellulose, polyvinylpyrrolidones and derivatives thereof, alginates, carrageenan, collagens, polyoxyethylene-polyoxypropylene copolymers, polyalkylene glycols, lecithins, phospholipids, pegylated phospholipids, gelatine, cholesterol, casein, tragacanth, pectins, gums, especially gum arabic, dextran, polyoxyethylen stearates, polyvinyl alcohol, polyethylene glycols, triethanolamine, tocopherol-polyethylene glycols and combinations thereof in an amount from 0.05% by weight to 15% by weight, based on the total weight of the composition, at least one active pharmaceutical ingredient selected from the group of avermectins in an amount from 2% by weight to 15% by weight, based on the total weight of the composition, having a mean particle size of 0.01 µm to 20 µm, and water in an amount from 70% by weight to 97.9% by weight, based on the total weight of the composition.

Preferably, the at least one active pharmaceutical ingredient selected from the group of avermectins is ivermectin.

Further, the aqueous composition is preferably for use in a pharmaceutical composition or medicament, wherein preferably for the use in the prevention or treatment of viral diseases, in particular for use in the prevention or treatment of a SARS-Covid-2 infection or of a HIV infection.

## Description

### TECHNICAL FIELD AND PRIOR ART

The present invention relates to a novel aqueous composition comprising avermectins in particular for nasal applications. The present invention also relates to a discharging device comprising this aqueous composition and to the use of this aqueous composition in a pharmaceutical composition or medicament. Further this system of aqueous composition and discharging device is particular useful in the treatment of viral diseases via a nasal application.

Avermectins have been found to be active against the SARS-Covid 2 virus. However, avermectins have a low solubility in aqueous systems and are sensitive to oxidation which may lead to impurities and by-products. To prevent oxidation oral dosage forms of avermectin often comprise strong oxidation inhibitors like Butylhydroxytoluol (Driponin^{®} 3mg) or Butylhydroxyanisol (Scabioral^{®}). However, these oxidation inhibitors show a low solubility in aqueous systems as well.

In addition to the low solubility and high oxidation sensitivity, avermectins possess a low permeability through organic tissue which impairs the release profile and bioavailability of avermectins. To improve the bioavailability of avermectins the application of a highly concentrated avermectin medication can be a solution. However, a high concentration of avermectins in an aqueous composition is nearly impossible to facilitate.

Therefore, an aqueous composition of avermectins cannot facilitate an acceptable solubility of avermectins and oxidation inhibitors in an aqueous composition for medical use and a suitable concentration of avermectins which is needed to assure a good bioavailability.

The use of surface modifiers and ionic surfactants has been described in detail already in US 5,298,262 A for the stabilisation of active pharmaceutical compounds in the form of nanoparticles. However, no avermectins have been used and also no intranasal applications have been described which need a careful and different design with regard to viscosity, mucoadhesion and applicability of such systems. The use of surface modifiers has been also described in US 2010/316725 A1. However, this publication is related to meloxicam nanoparticulate formulations intended for an injectable composition. US 2023/0028307 A1 is describing a method for treating a neurological disorder comprising the administration of a composition with an effective amount of avermectins to a subject in need of such treatment, but no SARS-Covid-2 treatment has been described nor any compositions for a nasal application. US 2023/0085607 A is describing a method of augmenting the prophylactic and/or therapeutic effects of ivermectin on COVID-19 when administering ivermectin together with one or more natural ingredient. However, no single administration or specific nasal application is described. US 2023/0087473 A describes the use of an anthelmintic drug like ivermectin and essential oil(s) in the form of a suspo-emulsion for naso-pulmonary use. However, no aqueous compositions are disclosed.

It is an object of the present invention to provide a novel aqueous composition comprising avermectins, which has superior properties with regard to solubility, stability and bioavailability of avermectins, compared to conventional compositions comprising avermectins, preferably suitable for a nasal administration.

### SUMMARY OF THE INVENTION

The above object and further objects are solved by an aqueous composition comprising avermectins according to claim 1, a discharging device according to claim 13 and an aqueous composition for use in a pharmaceutical composition or medicament according to claim 14 and claim 15. Preferred embodiments are defined in the dependent claims. Preferably, but not necessarily, said aqueous composition is especially suitable for a nasal administration. The wording of all claims is explicitly incorporated into the content of this description by reference.

The present invention provides an aqueous composition comprising or consisting of
- at least one surface modifier selected from a group consisting of cellulose derivatives, in particular hydroxypropylmethyl cellulose, methylcellulose and hydroxypropylcellulose, polyvinylpyrrolidones and derivatives thereof, alginates, carrageenan, collagens, polyoxyethylene-polyoxypropylene copolymers, polyalkylene glycols, lecithins, phospholipids, pegylated phospholipids, gelatine, cholesterol, casein, tragacanth, pectins, gums, especially gum arabic, dextran, polyoxyethylen stearates, polyvinyl alcohol, polyethylene glycols, triethanolamine, tocopherol-polyethylene glycols and combinations thereof in an amount from 0.05% by weight to 15% by weight, based on the total weight of the composition,
- at least one active pharmaceutical ingredient selected from the group of avermectins in an amount from 2% by weight to 15% by weight, based on the total weight of the composition, having a mean particle size of 0.01 µm to 20 µm, and
- water in an amount from 70% by weight to 97.9% by weight, based on the total weight of the composition.

Preferably the at least one surface modifier is a water-dispersible natural or synthetic polymer selected from the group of polymers consisting of cellulose derivatives, in particular hydroxypropylmethyl cellulose, methylcellulose and hydroxypropylcellulose, polyvinylpyrrolidones and derivatives thereof, alginates, carrageenan and polyoxyethylene-polyoxypropylene copolymers, polyalkylene glycols, pegylated phospholipids, tragacanth, pectins, gums, especially gum arabic, dextran, polyoxyethylen stearates, polyvinyl alcohol, polyethylene glycols, tocopherol-polyethylene glycols and combinations thereof.

Further preferred, the aqueous composition comprises only one surface modifier. Preferably the one surface modifier is selected from a group consisting of cellulose derivatives, in particular hydroxypropylmethyl cellulose, methylcellulose and hydroxypropylcellulose, polyvinylpyrrolidones and derivatives thereof, alginates, carrageenan, collagens, polyoxyethylene-polyoxypropylene copolymers, polyalkylene glycols, lecithins, phospholipids, pegylated phospholipids, gelatine, cholesterol, casein, tragacanth, pectins, gums, preferably gum arabic, dextran, polyoxyethylen stearates, polyvinyl alcohol, polyethylene glycols, triethanolamine and tocopherol-polyethylene glycols. Preferably the one surface modifier is present in the aqueous composition in an amount from 0.05% by weight to 15% by weight, based on the total weight of the composition.

It was found that the inventive aqueous composition is especially useful for providing a novel aqueous composition comprising avermectins, which has superior properties with regard to solubility, stability and bioavailability of avermectins, compared to conventional compositions comprising avermectins.

Usually, surface modifiers are combined with at least one surfactant, preferably two surfactants, and other additives in aqueous compositions to increase the stability of the composition. However, surprisingly, in the present invention it could be shown that the inventive composition, in particular due to the selection of the at least one surface modifier, the particle size of the pharmaceutical active ingredient and the selected weight of the ingredients, based on the total weight of the composition, as described above, can ensure sufficient stability of the composition, in particular solubility, stability and bioavailability of avermectins, without a surfactant or with a maximum of one surfactant.

In a preferred embodiment of the invention the inventive aqueous composition is sterile.

In a further preferred embodiment of the invention the inventive aqueous composition is free of a surfactant.

The terms used in the claims and in the overall description are defined as follows.

The term "aqueous composition" as used herein, means a composition which is free of organic solvents or non-aqueous components. Preferably, the aqueous composition is a suspension.

The term "free of organic solvents or non-aqueous components" as used herein means that less than 10% by weight, in particular less than 5% by weight, preferably less than 2% by weight, more preferably less than 2% by weight organic solvents or non-aqueous components are present in the aqueous composition, based on the total weight of the composition.

The term "surface modifier" as used herein, means a substrate, preferably a polymer, further preferred a water-soluble substrate, used in the process of pharmaceutically active compound delivery, which is adsorbed partially or completely on the active pharmaceutical ingredient, preferably on micro- or nanoparticles of the active pharmaceutical ingredient, and improves thereof the stability of the said active pharmaceutical ingredient in the aqueous composition.

The term "surfactant" as used herein, means a compound, preferably a water-soluble compound, that lowers the surface tension between two liquids or between a liquid and a solid in a composition, enabling or supporting the stabilization and wetting of the active pharmaceutical ingredient, in particular the stability of the multiparticulates of said active pharmaceutical ingredient in the aqueous composition.

The term "active pharmaceutical ingredient" (API) as used herein, means any substance or combination of substances used in a pharmaceutical composition or in a medicament or in a drug, intended to provide the desired pharmacological activity, preferably avermectins, more preferably ivermectin. In other words, the "active pharmaceutical ingredient" is the component in the pharmaceutical composition or medicament or drug that is biologically active. Other terms and expressions with the same meaning are "pharmaceutically active compound", "pharmaceutically active agent" or "pharmaceutically active ingredient".

The term "multiparticulate" as used herein, means a plurality of particles of at least one active pharmaceutical ingredient, wherein the particles are of the same size or of different size.

The term "sterile" as used herein, means free from living germs or microorganisms, in particular aseptic.

The term "mucoadhesion" as used herein, means an adhesion at organic tissues such as nose and mouth, which are mostly covered by mucus or mucous membranes.

A syringe is a reciprocating pump (including piston pump, plunger pump and diaphragm pump) consisting of a plunger/piston tightly fitting within a cylindrical tube. This construction allows the syringe to take in and to expel liquid (or gas) through a discharge orifice at one end of the tube.

A spray device is able to generate an aerosol, namely a suspension of fine liquid droplets in air or another gas, by a spray system with the means of atomization. Such spray devices are e.g. used in nasal sprays which deliver medication in the nasal cavities for local and/or systemic effects.

In preferred embodiments of the invention the aqueous composition can additionally comprise additives which can further optimize the properties of the inventive composition, e.g. its physical or chemical properties. Preferably these additives are present in the inventive composition in a total amount of less than 10 % by weight, in particular in a total amount of less than 5 % by weight. In this context it is even further preferred if the total amount of additives in the inventive aqueous composition does not exceed 3 % by weight.

Further, these additives can be taste masking excipients, preservatives, antioxidants, chelating agents and other additives which can be present in an aqueous composition for pharmaceutical use.

Taste masking additives are substances or chemicals which are added to improve the taste of desired compositions. According to the invention sugars or sugar alcohols can be used as taste masking agents.

Preservatives are substances or chemicals which are added to (among others) pharmaceutical compositions to prevent microbial growth or other undesired changes of the composition. According to the invention parabens and/or benzalkonium-chloride can be used as preservatives. Other examples for useful preservatives are sorbic acid, sodium sorbate and other sorbates, as well as benzoic acid and benzoates.

Antioxidants are compounds that inhibit oxidation, i.e. a chemical reaction which can produce free radicals. Said free radicals can lead to chain reactions which may damage the cells of an organism. In addition, antioxidants can further prevent oxidation of the at least one active pharmaceutical ingredient selected from the group of avermectins in the inventive aqueous composition and thereby further prevent impurities and by-products of avermectins which are sensitive to oxidation.

Antioxidants which can be used according to the invention are naturally occurring substances, like citric acid and ascorbic acid (vitamin C). A synthetic antioxidant is e.g. butylated hydroxytoluene (BHT).

Chelating agents are chemical compounds which form complexes with metal ions. They are used e.g. as additives to bind metal ions, in particular heavy-metal ions in the form of a complex (chelate). The chelating agents useful in binding metal ions, especially heavy-metal ions are known to a person skilled in the art. An important example for such a chelating agent is ethylenediaminetetraacetic acid (EDTA).

According to the invention, the at least one surface modifier is preferably present in the inventive aqueous composition in an amount from 0.05% by weight to 10% by weight, preferably from 0.1 % by weight to 10% by weight, more preferably in an amount from 0.1% by weight to 8% by weight, based on the total weight of the composition.

In a further embodiment of the invention, the at least one surface modifier is selected from a group consisting of alginate, phospholipid, in particular MPEG-2000-DSPE, polyvinylalcohol, polyvinylpyrrolidone, in particular PVP PF 12, tocopherol-polyethylene glycols (TPGS), hydroxypropylmethyl cellulose (HPMC), dextran and combinations thereof.

Preferably, the at least one surface modifier is alginate. Alginate has been found to regulate the stability of the at least one pharmaceutical active compound, the viscosity and the mucoadhesion of the inventive aqueous composition especially advantageous.

Further, the following combinations of two surface modifiers are preferred, alginate and phospholipid, in particular MPEG-2000-DSPE, alginate and polyvinylalcohol and alginate and polyvinylpyrrolidone, in particular PVP PF 12.

As defined above the at least one surface modifier present in the inventive aqueous composition improves the stability of a pharmaceutical active ingredient in aqueous compositions, in particular of a pharmaceutical active ingredient having a mean particle size of 0.01 µm to 20 µm.

Further, the surface modifiers mentioned above can particularly advantageously form viscous solutions in aqueous systems and thereby enable the adhesion of the inventive aqueous compositions to tissues as nose or buccal areas in the mouth (mucoadhesion), which is difficult due to the coverage of these tissues by a mucus layer or mucous membranes and their mucociliary clearance which make adhesion challenging.

A balance between viscosity and adhesion of such aqueous composition and sprayability with a spray device or applicability with a blow-fill-seal ampoule applications must therefore be carefully considered when choosing the surface modifier and the amount of the surface modifier in the inventive aqueous composition.

The surface modifiers according to the invention are toxicological proven and can e.g. be used for topical nasal applications.

It was found that the inventive aqueous composition is, because of the chosen surface modifiers and the chosen weight of the surface modifiers especially useful for providing a good mucoadhesion of the inventive aqueous composition which allows a prolonged bioavailability of a therapeutically effective amount of active pharmaceutical ingredients after administration, in particular after nasal administration, while additionally ensuring the stability of the composition, in particular solubility, stability and bioavailability of avermectins.

Further, the at least one surface modifier is preferably selected from a group consisting of grades of a polyvinylpyrrolidone polymer (PVP), namely PVP grades 12 PF, 17 PF, K25 and K30.

Further, the at least one surface modifier may be selected from a group consisting of a polyvinylpyrrolidone copolymer with vinylacetate (VA), in particular the copolymer PVP VA 64.

Further, the at least one surface modifier may be selected from a group of polyoxyethylene-polyoxypropylene copolymers called Poloxamers. Poloxamers are nonionic triblock copolymers of a hydrophobic chain of polyoxypropylene flanked by two hydrophilic chains of polyoxyethylene. Preferably the at least one surface modifier is a Poloxamer type 188 or type 407 copolymer.

Further, the at least one surface modifier is preferably selected from a group comprising polyoxyethylene-polyoxypropylene copolymers under the trademark Kolliphor^{®} (BASF). The most preferred surface modifiers are Kolliphor P188 (Poloxamer type 188 copolymer) and Kolliphor P407 (Poloxamer type 407 copolymer).

Preferably, the viscosity of the inventive aqueous composition at a temperature of approx. 20 °C - 35 °C is from 1 mPas to 1000 mPas, preferably from 1 mPas to 100 mPas, more preferably from 1 mPas to 50 mPas.

According to the invention viscosity preferably measured with a viscometer, in particular a rheometer. A rheometer usually is a laboratory device measuring flow characteristics of a liquid, suspension or slurry in response to applied forces. A common device type is a so-called shear rheometer, in which a shear stress is applied to the corresponding material. Devices which can be used to measure the viscosity of the non-aqueous compositions according to the invention are rheometers from Anton Paar Germany GmbH, Ostfildern, Germany (e.g. MCR 101).

According to the invention the at least one active pharmaceutical ingredient selected from the group of avermectins is preferably ivermectin.

Avermectins are a group of 16 macrocyclic lactone derivatives which are mainly known to treat parasitic worms and insect pests. These compounds and their chemical structures are known to a person skilled in the art.

An important representative of the compounds known as avermectins is ivermectin which can be preferably present in the aqueous composition according to the invention.

In a preferred embodiment of the invention the at least one active pharmaceutical ingredient from the group of avermectins has a (low) solubility in water at room temperature (22-26°C) of < 1 mg/ml, preferably < 0,1 mg/ml.

The low solubility of active pharmaceutical ingredients negatively affects the uptake of said ingredients into the body, in particular in the nasal mucosa of the body, after administration. As a consequence, a therapeutic effect may not be achieved by a single administration, but a local depot formulation having a high concentration of active pharmaceutical ingredients using the intrinsic low solubility of the active pharmaceutical ingredients in a suitable aqueous composition to obtain a prolonged activity of the ingredients over the desired time interval. This is achieved by the inventive aqueous composition as described above. The prolonged therapeutic effect has to be understood as the time needed for the active pharmaceutical ingredient to be fast released from the aqueous composition into the organic tissue to enable the prolonged local therapeutic effect of the adsorbed ingredients, especially the corresponding multiparticulates known for e.g. for nanoparticles consisting of PLGA particles loaded with a fluorescence marker in bovine nasal mucosa [Mohammed A. Albarki and Maureen D. Donovan, AAPS PharmSciTech , 06/2020].

According to the invention the at least one active pharmaceutical ingredient selected from the group of avermectins, in particular ivermectin, is preferably present in the composition in an amount from 2 % by weight to 12 % by weight, preferably from 2 % by weight to 10 % by weight, preferably in an amount from 2 % by weight to 8 % by weight, more preferably in an amount from 4 % by weight to 8 % by weight based on the total weight of the composition.

It was found that the inventive aqueous composition is, preferably due to the chosen surface modifiers, especially useful for providing a high concentration of avermectins in the aqueous composition improving the in vivo prolonged therapeutic effect of a therapeutically effective amount of the active pharmaceutical ingredient from the group of avermectins after administration (bioavailability), preferably at internal organs, in particular after nasal administration.

According to the invention the at least one active pharmaceutical ingredient present in the aqueous composition has a mean particle size of 0.01 to 20 µm, preferably of less than 0.5 µm, in particular of less than 0.5 µm, with a preferred lower limit of 0.1 µm. Thus, the mean particle size of the active pharmaceutical ingredient is in the submicron range (preferred mean size < 3 µm, i.e. < 3000 nm), or even in the nano range (preferred mean size < 0,5 µm, i.e. < 500 nm).

In another preferred embodiment of the invention the active pharmaceutical ingredient has a mean particle size of less than 10 µm, preferably of less than 5 µm, preferably with a lower limit of 0.1 µm, wherein a mean particle size from 1 µm to 5 µm, preferably from 1 µm to 4 µm, more preferably from 1 µm to 3 µm or 2 µm to 3 µm, is even further preferred. Thus, the mean particle size of the active pharmaceutical ingredient is preferably in the micron range.

In an embodiment of the invention the at least one active pharmaceutical ingredient present in the aqueous composition preferably consists of at least two groups of particles with different (mean) particle sizes. Preferably at least one of these groups (fractions) consists of particles with sizes in the micron range (preferred mean size < 3 µm, i.e. < 3000 nm) and the other group consists of particles with sizes in the submicron or nano range (preferred mean size < 0,5 µm, i.e. < 500 nm). Preferably the at least one active pharmaceutical ingredient from the group of avermectins is present in two groups of particles, namely in a mean particle size of 0.1 µm to 0.3 µm of one particle fraction (the submicron fraction) and a mean particle size of 1 to 10 microns of another particle fraction (the microparticle fraction).

The term "mean particle size" as used herein, preferably means a particle or 50 % of all particles of the at least one active pharmaceutical ingredient (D50) relates to a particle whose dimensions, in particular its diameter, preferably mean diameter, lies in a range from 0,01 to 20 µm for example, preferably in the ranges as defined above. In this context, the term "diameter" is to be understood in the case of a spherical particle to mean the diameter of the sphere, i.e. twice the radius of the sphere. In the case of a non-spherical particle, the term "diameter" is to be understood as the largest possible distance that two points along a circumference of the particle can take from each other.

The mean particle size distribution is preferably obtained by laser diffraction. In a laser diffraction measurement, a laser beam passes through a dispersed particulate sample and the angular variation in intensity of the scattered light is measured. Large particles scatter light at small angles relative to the laser beam and small particles scatter light at large angles. The angular scattering intensity data is then analyzed to calculate the size of the particles that created the scattering pattern using the Mie theory of light scattering. The particle size is reported as a volume equivalent sphere diameter. The folded optical design in the Mastersizer 3000 (from Malvern Panalytical), which was in particular used for the measurements, provides a particle size range from 10 nm up to 3.5 mm using a single optical measurement path. The Mastersizer 3000 uses a sequential combination of measurements with red and blue light sources to measure across the entire particle size range.

The above described mean particle size of the at least one active pharmaceutical ingredient from the group of avermectins was found to be especially useful to obtain a homogenous distribution of said ingredients in the inventive aqueous composition.

In an embodiment of the invention the desired particle size of the at least one active pharmaceutical ingredient has been preferably obtained by grinding, ball milling, wet bead milling, high pressure homogenization, homogenization, micronisation or a combination of at least two of the described methods.

In a further embodiment of the invention the at least one active pharmaceutical ingredient has been preferable stored in an inert container to avoid degradation after milling or grinding. In particular brown glass bottles closed with a tight seal and cap have been found out to be suitable to avoid significant degradation over storage time.

The particle size as defined above promotes an effect to obtain an increased biological activity (resorption) by improving the release profile and the bioavailability of hydrophobic particles, preferably hydrophobic active pharmaceutical ingredient particles, in the inventive aqueous composition and to increase the stability of hydrophobic particles, preferably hydrophobic active pharmaceutical ingredient particles, in the inventive aqueous composition.

Further, due to the above-described particle size of the at least one active pharmaceutical ingredient the prolonged therapeutic effect of the inventive composition into a body, after administration at organic tissues, is especially advantageous in comparison to other conventional compositions comprising active pharmaceutical ingredients. Due to the advantageous prolonged therapeutic effect of the inventive aqueous composition, lesser amounts of active pharmaceutical ingredients in the composition are needed to achieve the same therapeutical effect as conventional compositions.

According to the invention the aqueous composition can further comprise at least one buffer, wherein preferably the at least one buffer is present in an amount from 0.001% by weight to 10% by weight, preferably from 0.1 % by weight to 8 % by weight, more preferably from 0.14% by weight to 6% by weight, based on the total weight of the composition.

A buffer or buffer system is an aqueous (buffer) solution consisting of a mixture of a weak acid and its conjugate base, or vice versa. The pH value of this solution changes only little when a small amount of a strong acid or base is added.

A pH value of 5 to 8 has been found to be particularly advantageous, for the inventive aqueous composition, with regard to solubility, stability and bioavailability of avermectins, compared to conventional compositions comprising avermectins.

Preferably, no or only a small change in pH during the storage time of the inventive aqueous composition of 1, in particular 0.5 to 0.1, or less is achieved with the aqueous composition and can be further optimized with a buffer, and indicates a particularly stable inventive aqueous composition, which guarantees continuous solubility and bioavailability of avermectins in the composition. According to the invention the at least one buffer preferably comprises at least one inorganic salt or at least one organic compound. The buffers described below are especially useful for inhibiting the oxidation of the active pharmaceutical ingredient, in particular from the group of avermectins. Preferably the buffer contributes to an advantageous inhibition of oxidation of the active pharmaceutical ingredient from the group of avermectins in combination with the at least one surface modifier.

According to the invention the at least one buffer comprises a salt selected from a group of inorganic salts, wherein preferably the group of inorganic salts consist of monosodium phosphate, disodium phosphate, sodium bicarbonate, sodium hydrogencarbonate, dicalcium phosphate, tris(hydroxymethyl) aminomethane (TRIS), sodium citrate and sodium acetate and/or from a group of organic compounds consisting of glycine, gluconic acid and chelating agents, in particular ethylenediaminetetraacetic acid (EDTA).

In a preferred embodiment of the invention the at least one organic compound is selected from a group comprising glycine, gluconic acid and chelating agents, in particular ethylenediaminetetraacetic acid (EDTA).

In a more preferred embodiment of the invention the at least one buffer is a sodium citrate buffer combined with citric acid, which is also called citrate buffer in this application.

In another preferred embodiment of the invention the at least one buffer is sodium citrate combined with citric acid in a molarity of 1 mMol to 2 Mol, preferably 1 mMol to 1 Mol and more preferably of 5 mMol to 60 mMol, in particular of 5 mMol to 50 mMol.

According to the invention the aqueous composition may further comprise one surfactant selected from a group of ionic surfactants, consisting of sodium taurocholate (STC), sodium glycocholate (SGC), dioctyl sulfosuccinate ester (DOSS), bile salts and sodium dodecyl sulphate (SDS) or from a group of non-ionic surfactants consisting of polysorbate esters.

Preferably, the surfactant is present in the inventive composition in an amount from 0.1% by weight to 5% by weight, preferably from 0.1% by weight to 1% by weight, more preferably in an amount from 0.1% by weight to 0.5% by weight, based on the total weight of the composition.

The above mentioned amounts of the surfactant have been found to be particularly advantageous, because the inventive aqueous composition comprising the surfactant in amounts above 5 %, based on the total weight of the aqueous composition, no longer supports some of the advantages of the inventive aqueous composition and amounts above 7.5 % by weight, based on the total weight of the aqueous composition, no longer supports most of the advantages of the inventive aqueous composition since the aqueous composition can show a foam development, in particular when using polyoxyethylene-sorbitan-monooleat (Tween^{®} 80) or sodium glycocholate (SGC).

According to the invention the surfactant preferably comprises one ionic or non-ionic surfactant. The surfactants described below are especially useful for further improving the wettability and stabilization of the active pharmaceutical ingredient in the aqueous composition to ensure a homogeneous distribution of active pharmaceutical ingredients, preferably hydrophobic active pharmaceutical ingredient particles, in the inventive aqueous composition.

It was found that the inventive aqueous composition comprising a surfactant may be useful for further avoiding an oxidation of the active pharmaceutical ingredient, which may lead to impurities and by-products, and increasing the stability of the active pharmaceutical ingredient. Therefore, the use of antioxidants to prevent oxidation of active pharmaceutical ingredients, in particular from the group of avermectins, can be avoided even more secure or the amount of antioxidants in the aqueous composition can be reduced even more.

Among other advantages the use of one surfactant was found to be enough to contribute to the advantages described above.

In a further preferred embodiment of the invention the inventive aqueous composition is free of a second, in particular free of more than one, surfactant.

Usually at least one, preferably two surfactants, are used in aqueous compositions to increase the stability of the composition. However, surprisingly, in the present invention it could be shown that the composition, in particular due to the selection of the surface modifier and the choice of the weight, based on the total weight of the composition, as described above, can do without or with a maximum of one surfactant to ensure sufficient stability of the composition, in particular solubility, stability and bioavailability of avermectins.

In a further preferred embodiment of the invention the ionic surfactant is selected from a group comprising dioctyl sulfosuccinate ester, sodium dodecyl sulphate, sodium taurocholate (STC) and sodium glycocholate (SGC).

In a more preferred embodiment of the invention the ionic surfactant is sodium glycocholate (SGC).

In another preferred embodiment of the invention the non-ionic surfactant is selected from a group comprising polysorbate esters, e.g. polyoxyethylene-sorbitan-monolaurat (Tween^{®} 20), polyoxyethylene-sorbitan-monopalmitat (Tween^{®} 40), polyoxyethylene-sorbitan-monostearat (Tween^{®} 60), polyoxyethylene-sorbitan-tristearat (Tween^{®} 65) and polyoxyethylene-sorbitan-monooleat (Tween^{®} 80). Preferably the compounds are sold under the trademark Tween^{®} (Croda).

In a more preferred embodiment of the invention the non-ionic surfactant is polyoxyethylene-sorbitan-monooleat (Tween^{®} 80).

It was found that the inventive aqueous composition comprising one of the above mentioned surfactants might be even more useful for providing an in vivo prolonged therapeutic effect of a therapeutically effective amount of the active pharmaceutical ingredients, in particular from the group of avermectins, after administration (bioavailability), preferably at internal organs, in particular after nasal administration, and an advantageous stability of the active pharmaceutical ingredients, in particular from the group of avermectins.

According to the invention, the aqueous composition is preferably a prolonged release composition, which provides an equally, prolonged activity, of the active pharmaceutical ingredient, in particular activity in the organic tissue, in particular of a human body, over a period of at least 1 hour, more preferably of at least 2 hours after a single administration.

According to the invention an aqueous composition is preferred, wherein
- the at least one surface modifier is alginate in an amount from 0.05% by weight to 1.5% by weight, based on the total weight of the composition,
- the at least one active pharmaceutical ingredient selected from the group of avermectins is ivermectin, present in an amount from 2% by weight to 8% by weight, based on the total weight of the composition,
- ivermectin has a mean particle size from 0.01 µm to 10 µm,
- the aqueous composition comprises one surfactant which is polyoxyethylene-sorbitan-monooleat (Tween^{®} 80), present in an amount 0.1% by weight to 0.5% by weight, based on the total weight of the composition, and
- water is present in an amount from 90% by weight to 97.9% by weight, based on the total weight of the composition.

The aqueous composition mentioned above was found to be especially advantageous, concerning the adhesion properties and the viscosity properties of the inventive aqueous composition as well as the stability, solubility and uptake of an active pharmaceutical ingredient, especially with a mean particle size of 0.1 µm to 0.5 µm, from the inventive aqueous composition into the body providing a sustained release of the compound for a period of at least 3 days, preferably at least 5 days, after a single administration at organic tissue (bioavailability).

According to the invention an aqueous composition is preferred, wherein
- the at least one surface modifier is alginate in an amount from 0.05% by weight to 1.5% by weight, based on the total weight of the composition,
- the at least one active pharmaceutical ingredient selected from the group of avermectins is ivermectin, present in an amount from 2% by weight to 8% by weight, based on the total weight of the composition,
- ivermectin has a mean particle size from 0.01 µm to 10 µm,
- the aqueous composition comprises one surfactant which is polyoxyethylene-sorbitan-monooleat (Tween^{®} 80), present in an amount 0.1% by weight to 0.5% by weight, based on the total weight of the composition,
- water is present in an amount from 84% by weight to 97.7% by weight, based on the total weight of the composition,
- and the buffer present in the aqueous composition is present in an amount from 0,14% by weight to 6% by weight, based on the total weight of the composition and comprises sodium citrate.

The aqueous composition mentioned above was found to be especially advantageous, concerning the adhesion properties and the viscosity properties of the inventive aqueous composition as well as the stability, solubility and uptake of an active pharmaceutical ingredient, especially with a mean particle size of 1 µm to 4 µm, preferably 1 µm to 3 µm, from the inventive aqueous composition into the body providing a sustained release of the compound for a period of at least 3 days, preferably at least 5 days, after a single administration at organic tissue (bioavailability).

It was found that the inventive aqueous composition comprising at least one buffer, in particular one buffer, is especially useful for providing a good stability of the active pharmaceutical ingredient having a mean particle size in the micron range, in particular a mean particle size of 1 µm to 4 µm, preferably 1 µm to 3 µm.

According to the invention the rest of the aqueous composition not being a surface modifier, surfactant, active pharmaceutical ingredient, additives and optionally a buffer is preferably water, in particular demineralized water or purified water or even water for injection.

In another embodiment of the invention water is present in the inventive aqueous composition in an amount from at least 60% by weight to 97.9% by weight, preferably from 80% by weight to 94% by weight, more preferably in an amount from 80% by weight to 90% by weight, based on the total weight of the composition.

With respect to further features and advantages of the above described aqueous composition, reference is made in its entirety to the previous description.

According to the invention the aqueous composition is preferably provided for nasal application, in particular in the form of a nasal spray.

Due to the low solubility of avermectins in aqueous media a multiparticulate inventive aqueous composition as described above was found to show an advantageous consistent application of suitable aerosolized droplets in an appropriate size range, avoiding loss of drug medication from the nasal cavity after application (to throat and/or lung) and avoiding clogging of the spray pump device by using an increased drug loading in the aqueous composition.

The invention further provides a discharging device, in particular a blow-fill-seal-ampoule, syringe or a spray device, wherein the discharging device is filled with the aqueous composition as claimed and as defined above.

In another embodiment of the invention the discharging device is preferably a CycloOlefinPolymer (COP) syringe, which shows the advantage of bearing no risk to have any residual glass particles from the manufacturing process included.

In another embodiment of the invention the discharging device is preferably a plastic spray device, suitable for application of aqueous compositions.

Finally, the invention provides an aqueous composition as claimed and as defined above, for use in a pharmaceutical composition or a medicament, wherein preferably said use is for the prevention or treatment, in particular local treatment, of submicroscopic infectious agents that replicates only inside the living cells of an organism, so-called viral infections more preferably in the prevention or treatment of a SARS-Covid-2 infection or of a HIV infection or of a dengue virus infection.

When infected, a host cell is forced to rapidly produce thousands of identical copies of the original virus. Ivermectin has found to be active against a viral disease, the SARS-Covid 2 virus [https://www.monash.edu/discovery-institute/news-and-events/news/2020-articles/Lab-experiments-show-anti-parasitic-drug,-Ivermectin,-eliminates-SARS-CoV-2-in-cells-in-48-hours].

The inventive aqueous composition containing multiparticulates of avermectins, in particular ivermectin, has the advantage of killing virus particles locally entering the nasal cavity from the environment due to the advantageous therapeutical effect comprised by a high concentration of the at least one active pharmaceutical ingredient selected from the group of avermectins and a large surface of the multiparticulates of the active pharmaceutical ingredient.

The inventive aqueous composition can be applied in volumes from 20-400 µl, preferably from 100-200 µl, in particular into the nasal cavity.

In a further embodiment of the invention the selected dose for a nasal treatment of a SARS-Covid-2 infection is equal or above 3 mg per nostril for ivermectin in humans.

With respect to further features and advantages of the use, in particular in terms of the aqueous composition, reference is made in its entirety to the previous description.

Further features and advantages of the invention will become clear from the following examples in conjunction with the subject-matter of the dependent claims. The individual features can be realized either singularly or in combination in one embodiment of the invention. The preferred embodiments merely serve for illustration and better understanding of the invention and are not to be understood as in any way limiting the invention.

### EXAMPLES

The figures show the following:
Figure 1:
   graphically shows particle size distribution (PSD) of batch 004-SUS-G-2212-019-01 (initial). Parameter are as follows: Particle name: Ivermectin, Particle refractive index: 1.600, Particle absorption value: 0.050, Name Dispersing medium: Water, Refractive index Dispersing medium: 1.330, Scattering model: Mie, Analysis model: Universal, Weighted deviation: 1, 81 %, Laser shading: 4.48 %, Light shading - blue: 5.88 %, Stirrer speed: Reached 1500 rpm, Concentration 0.0010 %, Specific surface area: 3064 m²/kg, Uniformity: 1.133, Width: 2.789, Dv(10): 0.898 pm, Dv(50): 2.56 pm, Dv:(90) 8.05 pm.
Figure 2:
   graphically shows the zetapotential of batch 004-SUS-G-2212-019-01.
Figure 3:
   graphically shows particle size distribution (PSD) of batch F004-SUS-G-2212-018-01. Parameter are as follows: Particle name: Ivermectin, Particle refractive index: 1.600, Particle absorption value: 0.050, Name Dispersing medium: Water, Refractive index Dispersing medium: 1.330, Scattering model: Mie, Analysis model: Universal, Weighted deviation: 1, 79 %, Laser shading: 4.45 %, Light shading - blue: 5.77 %, Stirrer speed: Reached 1500 rpm, Concentration 0.0012 %, Specific surface area: 2697 m²/kg, Uniformity: 3.899, Width: 16.776, Dv(10): 0.990 pm, Dv(50): 2.84 pm, Dv:(90) 48.7 pm.
Figure 4:
   graphically shows the zetapotential of batch F004-SUS-G-2212-018-01.

### Example 1: Preparation of multiparticulates of ivermectin via dry milling/grinding

The pharmaceutically active agent is grinded in a mortar/pestle system to the appropriate particles size prior to its use. Afterwards the Particles Size Distribution (PSD) is measured using a Malvern Master Sizer 3000. The samples after grinding are stored in tightly closed brown glass bottles.

### Example 2: Preparation of multiparticulates of ivermectin via dry milling/micronisation

The pharmaceutically active agent is micronised to the desired particles size using a jet mill (e.g. from SpiraNo 100 from MCKO, Germany) and screw dosing system (e.g. ZD 9 from Three-Tec Ltd., Seon, Switzerland). The diameter of the injection nozzle is selected at about 6 mm, the milling gas pressure about 2 bar, the milling temperature is about 18 °C. The duration of the process is about 5-10 minutes depending on the particle size of the non-micronised active agent. Afterwards the PSD is measured with a Malvern Master Sizer 3000. The samples after micronisation are stored in tightly closed brown glass bottles.

### Example 3: Preparation of a multiparticulate aqueous suspension of ivermectin via wet milling

The grinded pharmaceutically active agent (according to procedure 1 or 2) is weighed into a 2 ml PP (Polypropylene) tube containing about 1 g milling beads (made of yttrium stabilised zirconium oxide or comparable) of appropriate size (e.g. 0.1 mm - 0.8 mm). The aqueous solution comprising the at least one surface modifier with one surfactant and without a surfactant in the specified amounts as stabilisers for the multiparticulate suspensions are added and the PP tube is closed. The content is mixed for some minutes and placed into a dual centrifugation system (Delta Vita 1, Netzsch, Selb, Germany or comparable). The system is started and operated under the following conditions: 1500 rpm, 1.5 hours, active cooling (≤ 25 C). Afterwards the probes are stored overnight in order to settle eventually formed foam. The content from the PP tubes is withdrawn via a 1 ml syringe with 22-gauge needle, in order to separate the sample from the milling beads.

Afterwards the PSD is measured with a Malvern Master Sizer 3000. In addition, the Zetapotential, an index for the stability of the multiparticulate suspensions is measured using a Zeta Sizer (e.g. Nano ZS) from Malvern Instruments (Dynamic Laser diffraction).

### Example 4: Preparation of an aqueous composition containing ivermectin multiparticulates via wet milling

The pharmaceutically active agent is grinded/micronised (see examples 1 and 2 above). Afterwards the grinded or micronised pharmaceutically active agent is suspended in the aqueous solution comprising the excipients (e.g. at least one surface modifier) in the specified amounts under stirring and homogenisation (Becomix or Ultraturrax). The stirring is performed at 2-25 °C until a homogeneous microsuspension is obtained.

Optionally, the named aqueous solution can be filtrated using a 0.22 µm pore size filter (e.g. Sartorius Sartopore 2, Capsule PES) prior to addition of the active ingredient in order to reduce the bioburden.

The suspension is then milled using a nanomill (e.g. using a VMA Getzmann, Germany (type SL 50 or comparable), in which prior to the bead milling the milling chamber (approx. 500 ml) is loaded with milling beads (made of yttrium stabilised zirconium oxide or comparable) of appropriate size (e.g. 0.1 mm - 0.8 mm). The (nano)milling process is conducted in a recirculation mode until the PSD matches the anticipated range with a mean value of about 0.1-0.5 µm. Once the anticipated PSD is reached, the process is stopped.

### Example 5: Preparation of an aqueous composition containing ivermectin multiparticulates - bulk microsuspension

The pharmaceutically active agent is grinded/micronised (see examples 1 and 2 above). Afterwards the grinded or micronised pharmaceutically active agent is suspended in the aqueous solution comprising the excipients (e.g. surface modifiers) in the specified amounts under stirring and homogenisation (Becomix or Ultraturrax). The stirring is performed at 2-25 °C until a homogeneous microsuspension is obtained.

Optionally, the named aqueous solution can be filtrated using a 0.22 µm pore size filter (e.g. Sartorius Sartopore 2, Capsule PES) prior to addition of the active pharmaceutical ingredient in order to reduce the bioburden.

### Example 6: Filling of suspensions obtained from procedure 4 and 5 into appropriate devices - final product

The resulting nanosuspensions (example 4) or microsuspension (example 5) are diluted with water or the selected buffer(s) to receive the final product. Afterwards the final product is filled (e.g. with a Bausch filling system Base type 533) into plastic or glass bottles (e.g. 10 ml brown glass bottle from Nipro, hydrolytic class I) and closed with pump heads, depending on the selected device (e.g. Aptar CPS spray pump). Optionally nitrogen is used in this step as inert gas.

### Example 7:

In accordance to the procedure described for example 5, a solution was prepared by dissolving 0.3 % Polysorbate 80 (Tween 80), 0.1% Sodium alginate and 89.6% Water. Afterwards 5% Ivermectin was suspended in this solution and finally 5% Citrate buffer (1 Molar) was added (batch F004-SUS-G-2212-019-01). Finally, the resulting suspension was homogenised. After preparation the PSD, pH and Zetapotential have been measured.

**Table 1: PSD, pH and Zetapotential of batch F004-SUS-G-2212-019-01**

| **Batch no. F004-SUS-G-** | **pH** | **PSD [µm]** | **PSD [µm]** | **PSD [µm]** | **PSD [µm]** | **Zeta potential [mV]** |
|---|---|---|---|---|---|---|
| | | **initial** | **After 2 weeks** | **after 3 weeks** | **After 4 weeks** | **initial** |
| 2212-019-01 | 6.7 | D10: 0.90 | D10: 0.92 | D10: 0.90 | D10: 0.90 | - 18.9 |
| | | D50: 2.56 | D50: 2.65 | D50: 2.56 | D50: 2.59 | |
| | | D90: 8.05 | D90: 11.4 | D90: 10.4 | D90: 11.8 | |

- D90: D90 means that 90% of the total particles are smaller than this diameter.
- D50: D50 means that 50% of the total particles are smaller than this diameter.
- D10: D10 means that 10% of the total particles are smaller than this diameter.

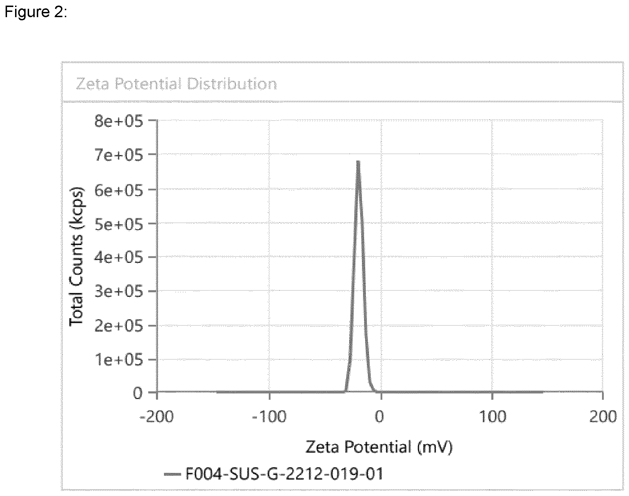

**Table 2: Zetapotential of batch 004-SUS-G-2212-019-01 according to Figure 2**

| Name | Mean | Standard Deviation | RSD | Minimum | Maximum | |
|---|---|---|---|---|---|---|
| Zeta Potential (mV) | -18,86 | - | - | -18,86 | -18,86 | |
| Zeta Peak 1 Mean (mV) | -18,86 | - | - | -18,86 | -18,86 | |
| Conductivity (mS/cm) | 0,1615 | - | - | 0,1615 | 0,1615 | |
| Wall Zeta Potential (mV) | -20,01 | - | - | -20,01 | -20,01 | |
| Zeta Deviation (mV) | 3,885 | - | - | 3,885 | 3,885 | |
| Derived Mean Count Rate (kcps) | 2.336E+06 | - | - | 2.336E+06 | 2.336E+06 | |
| Reference Beam Count Rate (kcps) | 2275 | - | - | 2275 | 2275 | |
| Quality Factor | 2,83 | - | - | 2,83 | 2,83 | |

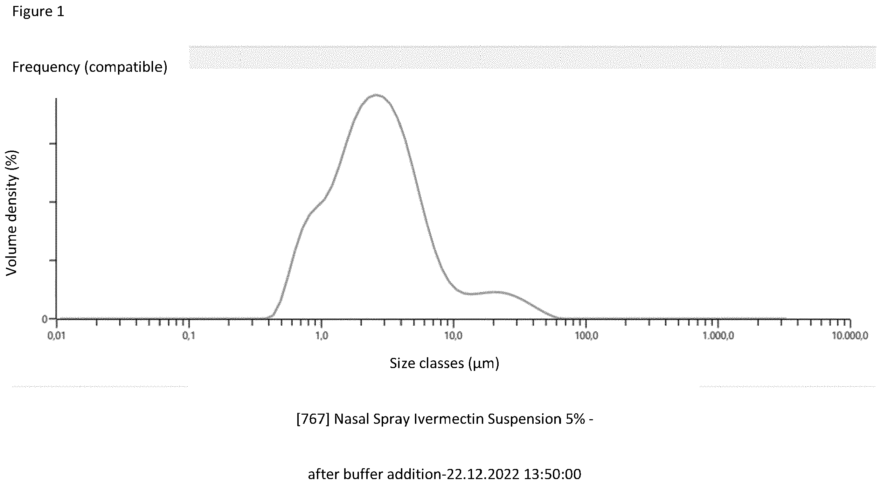

Table 1 shows, that the composition has a stable mean particle size distribution (D50) over a period of 4 weeks. The PSD measurement can also be seen in Figure 1. Further, Figure 2 and Table 2 shows, that the composition has strong negative zetapotential showing the good repulsion charge of the particles for the composition suitable for a stable microsuspension.

### Example 8:

In accordance to the procedure described for example 5, a solution was prepared by dissolving 0.3 % Polysorbate 80 (Tween 80), 2.0% HPMC and 87.7% Water. Afterwards 5% Ivermectin was suspended in this solution and 5% Citrate buffer (1 Molar) was added (batch F004-SUS-G-2212-018-01). Finally, the resulting suspension was homogenised. After preparation the PSD, pH and Zetapotential have been measured.

**Table 3: PSD, pH and Zetapotential of batch F004-SUS-G-2212-018-01**

| **Batch no. F004-SUS-G-** | **pH** | **PSD [µm]** | **PSD [µm]** | **PSD [µm]** | **PSD [µm]** | **Zetapotential [mV]** |
|---|---|---|---|---|---|---|
| | | **initial** | **After 2 weeks** | **after 3 weeks** | **After 4 weeks** | **initial** |
| 2212-018-01 | 6.7 | D10: 0.99 | D10: 1.42 | D10: 1.44 | D10: 1.46 | - 22.0 |
| | | D50: 2.84 | D50: 2.78 | D50: 2.87 | D50: 2.88 | |
| | | D90: 48.70 | D90: 17.46 | D90: 31.60 | D90: 27.80 | |

- D90: D90 means that 90% of the total particles are smaller than this diameter.
- D50: D50 means that 50% of the total particles are smaller than this diameter.
- D10: D10 means that 10% of the total particles are smaller than this diameter.

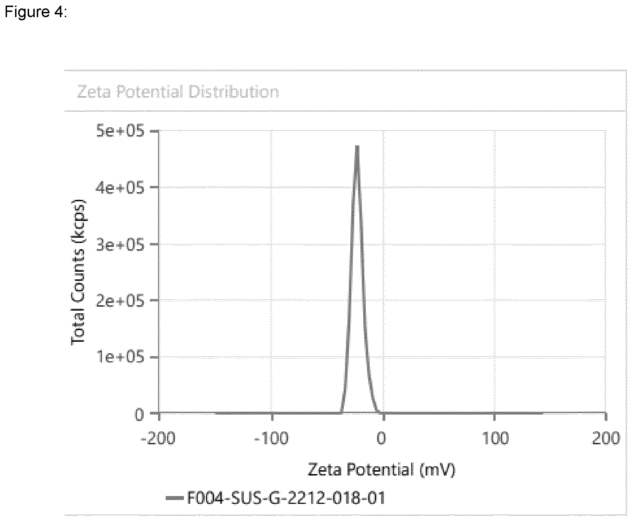

**Table 4: Zetapotential of batch F004-SUS-G-2212-018-01 according to Figure 4**

| Name | Mean | Standard Deviation | RSD | Minimum | Maximum |
|---|---|---|---|---|---|
| Zeta Potential (mV) | -21,96 | - | - | -21,96 | -21,96 |
| Zeta Peak 1 Mean (mV) | -21,96 | - | - | -21,96 | -21,96 |
| Conductivity (mS/cm) | 0,1513 | - | - | 0,1513 | 0,1513 |
| Wall Zeta Potential (mV) | -17,54 | - | - | -17,54 | -17,54 |
| Zeta Deviation (mV) | 5,149 | - | - | 5,149 | 5,149 |
| Derived Mean Count Rate (kcps) | 2,574E+06 | - | - | 2,574E+06 | 2,574E+06 |
| Reference Beam Count Rate (kcps) | 2228 | - | - | 2228 | 2228 |
| Quality Factor | 3,514 | - | - | 3,514 | 3,514 |

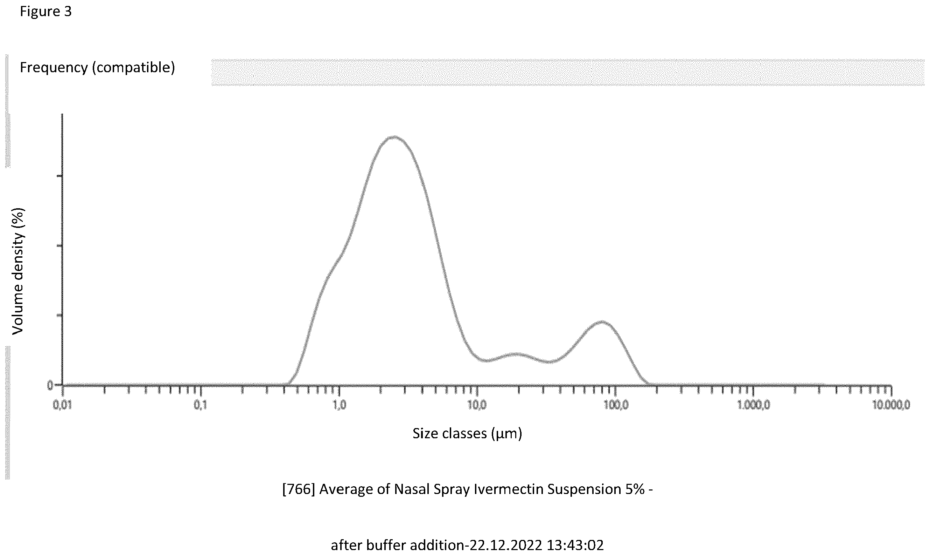

Table 3 shows, that the composition has a stable mean particle size distribution (D50) over a period of 4 weeks. The PSD measurement can also be seen in Figure 3. Further, Figure 4 and Table 4 shows, that the composition has strong negative zetapotential showing the good repulsion charge of the particles for the composition suitable for a stable microsuspension.

### Particle size distribution (PSD)

In some embodiments, the PSD of the pharmaceutically active agent, provided for the preparation of its multiparticulate suspension according to example 1 and example 2 (grinded/micronised), was measured using a Malvern Mastersizer 3000 (in water as dispersion medium, stirrer speed 1500 rpm, Mie presentation). The measurements have been made at ambient temperature.

### pH

In some embodiments, the pH value was measured for the aqueous compositions by using a pH meter (Mettler Toledo), type SevenExcellence.

### Zetapotential measurement

The measurement has been performed on Zetasizer from Malvern with a disposable capillary cell (DTS1070) at a temperature of approx. 25 °C, as dispersant water has been used with a refractive index of 1.330 and an equilibration time of 120s.

## Claims

1. Aqueous composition comprising
- at least one surface modifier selected from a group consisting of cellulose derivatives, in particular hydroxypropylmethyl cellulose, methylcellulose and hydroxypropylcellulose, polyvinylpyrrolidones and derivatives thereof, alginates, carrageenan, collagens, polyoxyethylene-polyoxypropylene copolymers, polyalkylene glycols, lecithins, phospholipids, pegylated phospholipids, gelatine, cholesterol, casein, tragacanth, pectins, gums, especially gum arabic, dextran, polyoxyethylen stearates, polyvinyl alcohol, polyethylene glycols, triethanolamine, tocopherol-polyethylene glycols and combinations thereof in an amount from 0.05% by weight to 15% by weight, based on the total weight of the composition,
- at least one active pharmaceutical ingredient selected from the group of avermectins in an amount from 2% by weight to 15% by weight, based on the total weight of the composition, having a mean particle size of 0.01 µm to 20 µm, and
- water in an amount from 70% by weight to 97.9% by weight, based on the total weight of the composition.

2. Aqueous composition according to claim 1, wherein the at least one surface modifier is present in the composition in an amount from 0.05% by weight to 10% by weight, preferably from 0.1 % by weight to 10% by weight, more preferably in an amount from 0.1% by weight to 8% by weight, based on the total weight of the composition.

3. Aqueous composition according to claim 1 or claim 2, wherein the at least one surface modifier is selected from a group consisting of alginate, phospholipid, polyvinylalcohol, polyvinylpyrrolidone, tocopherol-polyethylene glycols (TPGS), hydroxypropylmethyl cellulose (HPMC), dextran and combinations thereof.

4. Aqueous composition according to anyone of the preceding claims, wherein the at least one active pharmaceutical ingredient selected from the group of avermectins is ivermectin.

5. Aqueous composition according to anyone of the preceding claims, in particular according to claim 4, wherein the at least one active pharmaceutical ingredient selected from the group of avermectins is present in the composition in an amount from 2% by weight to 12% by weight, preferably from 2% by weight to 10% by weight, more preferably in an amount from 2% by weight to 8% by weight, based on the total weight of the composition.

6. Aqueous composition according to anyone of the preceding claims, wherein the at least one active pharmaceutical ingredient has a mean particle size of less than 0.5 µm, in particular from 0.1 µm to 0.5 µm and/or the at least one active pharmaceutical ingredient has a mean particle size of less than 10 µm, in particular from 1 µm to 5 µm, wherein a mean particle size from 1 µm to 3 µm is even further preferred.

7. Aqueous composition according to anyone of the preceding claims, wherein the aqueous composition further comprises at least one buffer, wherein preferably the at least one buffer is present in an amount from 0.001% by weight to 10% by weight, preferably from 0.1% by weight to 8% by weight, more preferably from 0.14% by weight to 6% by weight, based on the total weight of the composition.

8. Aqueous composition according to claim 7, wherein the at least one buffer comprises a salt selected from a group of inorganic salts consisting of monosodium phosphate, disodium phosphate, sodium bicarbonate, dicalcium phosphate, tris(hydroxymethyl) aminomethane (TRIS), sodium citrate and sodium acetate and/or from a group of organic compounds consisting of glycine, gluconic acid and chelating agents, in particular ethylenediaminetetraacetic acid (EDTA).

9. Aqueous composition according to anyone of the preceding claims, wherein the aqueous composition further comprises one surfactant selected from a group of ionic surfactants, consisting of sodium taurocholate (STC), sodium glycocholate (SGC), dioctyl sulfosuccinate ester (DOSS) and sodium dodecyl sulphate (SDS) or from a group of non-ionic surfactants consisting of polysorbate esters in an amount from 0.1% by weight to 5 % by weight, based on the total weight of the composition.

10. Aqueous composition according to anyone of the preceding claims, wherein
- the at least one surface modifier is alginate in an amount from 0.05% by weight to 1.5% by weight, based on the total weight of the composition,
- the at least one active pharmaceutical ingredient selected from the group of avermectins is ivermectin, present in an amount from 2% by weight to 8% by weight, based on the total weight of the composition,
- ivermectin has a mean particle size from 0.01 µm to 10 µm,
- the one surfactant present in the aqueous composition is polyoxyethylene-sorbitan-monooleat (Tween^{®} 80), present in an amount 0.1% by weight to 0.5% by weight, based on the total weight of the composition, and
- water is present in an amount from 90% by weight to 97.9% by weight, based on the total weight of the composition.

11. Aqueous composition according to claim 10, wherein a buffer is present in the aqueous composition in an amount from 0,14% by weight to 6% by weight, based on the total weight of the composition and comprises sodium citrate.

12. Aqueous composition according to anyone of the preceding claims, wherein said aqueous composition is provided for nasal application, in particular in the form of a nasal spray.

13. Discharging device, in particular a syringe or a spray device, wherein the discharging device is filled with the aqueous composition according to any one of claims 1 to 11.

14. Aqueous composition according to any one of claims 1 to 11, for use in a pharmaceutical composition or medicament, preferably for the use in the prevention or treatment of disorders or diseases via nasal administration, in particular for the prevention or treatment of viral diseases.

15. Aqueous composition according to any one of claims 1 to 12, for use according to claim 14 in the prevention or treatment of a SARS-Covid-2 infection.
